# EUROPEAN PATENT SPECIFICATION

(11) **EP 0 535 446 B1**
(45) Date of publication and mention of the grant of the patent: **02.12.1998**
(21) Application number: 92115818.4
(22) Date of filing: 16.09.1992
(51) Int. Cl.: A61K 31/71, A61K 31/355

(54) **Pharmaceutical compositions for topical use containing a chelating agent, a tocopheral and an antimicrobial agent**
Pharmazeutische Zusammensetzungen zur topischen Anwendung die ein Chelatierungsmittel, ein Tocopherol und einen antimikrobiellen Wirkstoff enthalten
Compositions pharmaceutiques à usage topique contenant un agent de chelation, un tocophérol et un agent antimicrobien

(30) Priority: 30.09.1991 IT RM910730
(43) Date of publication of application: 07.04.1993
(73) Proprietor: BONISCONTRO E GAZZONE S.r.l., I-00156 Roma (IT)
(72) Inventor: Bertone, Evaristo, I-00156 Roma (IT)
(74) Representative: Minoja, Fabrizio, Dr.

(56) References cited:
- WORLD PATENTS INDEX LATEST, section Ch, week 8520, class D, accession no. 85-118814, Derwent Publications Ltd, London, GB; & JP-A-60 058 500 (KANEBO K.K.) 04-04-1985

## Description

The present invention refers to pharmaceutical compositions for topical use useful for the therapy of skin ulcers, wounds, dermatoses, cutaneous infections and similar conditions.

It is known that the pharmacological treatment of certain dermatological pathologies, such as those mentioned above, is usually difficult and problematic and there is an urgent need for new and improved drugs able to induce or assist the healing process.

It has now been found that pharmaceutically compositions containing as active principles: a) a chelating agent, b) a tocopherol which could be vitamin E (α-tocopherol), c) an aminoglycoside antibiotic selected from gentamicin in concentration from 0.1 to 1% w/w or netilmicine or amikacine in concentrations from 0.5 to 8%, , when topically administered, are particularly effective for the treatment of pathological conditions in which a radical-scavenging activity, combined with antibacterial and antiinflammatory activity, is required.

The free oxygen-radicals, by means of peroxidation reactions, turn out to be particularly aggressive towards the cell membranes. The radicals are formed in vivo both during the usual cellular process and mainly by the action of phagocytes which are always present in any inflammatory condition, acute or chronic.

The compositions of the invention are characterized by the combination of an antioxidant, radical scavenging compound belonging to the class of tocopherols, with a chelating agents and an antimicrobial agent.

These components have unexpected synergistic effects.

The tocopherols may be selected from α-tocopherol or mixed tocopherols.

As a chelating agent, ethylenediaminotetraacetic acid (EDTA) or pharmaceutically acceptable salts thereof, dimercaprole disodium, deferoxamine, may be used.

The antimicrobial agent is selected from aminoglycoside antibiotics (amikacine, gentamicine, kanamycine, netilmicine). The use of amikacine is particularly preferred.

As a chelating agent, the use of EDTA or salts thereof is particularly preferred.

The chelating agent may be present in the compositions of the invention in concentrations ranging from 0.05 to 1% w/w; the tocopherols in concentrations ranging from 0.5 to 15% w/w whereas the amount of antibacterial agent will depend on the potency of the same. For instance, gentamicin may be present in concentrations from 0.1 to 1% w/w whereas metilmicine or amikacine may be present in concentrations from 0.5 to 8%.

The composition may of course comprise the usual excipients or carriers suited for the topical use, such as buffers, stabilizers (e.g. sulfites, bisulfites), thickening agents, preserving agents and the like. Typical pharmaceutical forms include powders, ointments, creams, lotions, gels. Powders are particularly advantageous in view of their drying action-hindering the maceration.

The invention is further illustrated by the following examples.

### EXAMPLE 1

100 g of cream contain:

| | | |
|---|---|---|
| Sodium edetate | g | 0.200 |
| Mixed concentrated tocopherols | g | 1.500 |
| Amikacine sulfate corresponding to amikacine base | g | 5.000 |
| Glycine | g | 0.100 |
| Cysteine hydrochloride | g | 0.100 |
| Methyl p-hydroxybenzoate | g | 0.090 |
| Propyl p-hydroxybenzoate | g | 0.010 |
| Hydroxypropylcellulose | g | 2.500 |
| Sorbitan monopalmitate | g | 1.430 |
| Polysorbate 80 | g | 0.570 |
| Purified water q.s. to | g | 100 |

### EXAMPLE 2

100 g of powder contain:

| | | |
|---|---|---|
| Sodium edetate | g | 0.200 |
| Mixed concentrated tocopherols | g | 1.500 |
| Amikacine sulfate corresponding to amikacine base | g | 5.000 |
| Precipitated silica | g | 3.000 |
| Zinc oxide | g | 5.000 |
| Magnesium carbonate | g | 7.000 |
| Cysteine | g | 0.100 |
| Talc q.s. to | g | 100 |

### EXAMPLE 3

### Clinical evaluation of the composition of Example 1

The composition of example 1 was clinically tested in several dermatological disease.

Table 1 shows the results, wherein the microbiological controls are also reported

**TABLE 1**

| Dermatological affection | Observation (n.) | Recovery (%) | Healing time (weeks) | Culture inhibition | Side effects |
|---|---|---|---|---|---|
| Varicose ulcer | -- | -- | -- | -- | none |
| Dermatological | -- | -- | -- | -- | none |
| lesion | | | (dry) | | |
| Eschars | -- | -- | -- | -- | none |
| Burns | -- | -- | -- | -- | 1 doubtful |

The above results prove that the pharmaceutical composition is effective, particularly for a fast epidermalization, an effective asepsys, an exiccative activity that avoid maceration, without causing side effects.

### Sensitization

The sensitizing properties of the composition according to example 1 were determined by the Magnusson and Klingmans's test, which is the most accurate and sensitive for this kind of investigations. The test was carried out on guinea pigs.

Table 2 shows that no potential risk of sensitization occurred when the above composition was used.

**TABLE 2**

| Concentrations | | | Results | |
|---|---|---|---|---|
| Induction phase | | Rising phase | Treated animals | Controls |
| Subcutaneous | Epicutaneous | | | |
| 0.2% | 0.5% | 1% | 0/20 | 0/20 |
| 0.4% | 1% | 1% | 0/20 | 0/20 |

## Claims

1. Topical pharmaceutical compositions containing as active principles:
a) a chelating agent
b) a tocopherol
c) an aminoglycoside antibiotic selected from gentamicin in concentration from 0.1 to 1% w/w or netilmicine or amikacine in concentrations from 0.5 to 8%.

2. Topical pharmaceutical compositions according to claim 1 characterized in that the chelating agent is selected from edetic acid or salts thereof, deferoxamine and dimercaprole (BAL).

3. Topical pharmaceutical compositions according to claim 1 or 2 wherein the chelating agent is present in concentrations ranging from 0.05 to 1% w/w.

4. Topical pharmaceutical compositions according to any one of the previous claims characterized in that the tocopherol is selected from Vitamine E (α-tocopherol) or mixed tocopherols.

5. Topical pharmaceutical compositions according to claim 4 wherein the tocopherols are present in concentrations ranging from 0.5 to 15%.

## Patentansprüche

1. Topische pharmazeutische Zusammensetzungen, enthaltend als aktive Wirkstoffe:
a) ein Chelatisierungsmittel
b) ein Tocopherol
c) ein Aminoglycosid-Antibiotikum, ausgewählt aus Gentamicin in einer Konzentration von 0,1 bis 1 Gew.-% oder Netilmicin oder Amikacin in Konzentrationen von 0,5 bis 8 %.

2. Topische pharmazeutische Zusammensetzungen entsprechend Anspruch 1, dadurch charakterisiert, daß das Chelatisierungsmittel ausgewählt wird aus Ethylendiamintetraessigsäure oder deren Salzen, Deferoxamin und Dimercaprol (BAL).

3. Topische pharmazeutische Zusammensetzungen nach Anspruch 1 oder 2, wobei das Chelatisierungsmittel in Konzentrationen im Bereich von 0,05 bis 1 Gew.-% vorliegt.

4. Topische pharmazeutische Zusammensetzungen nach einem der vorstehenden Ansprüche, dadurch charakterisiert, daß das Tocopherol ausgewählt wird aus Vitamin E (α-Tocopherol) oder gemischten Tocopherolen.

5. Topische pharmazeutische Zusammensetzungen nach Anspruch 4, wobei die Tocopherole in Konzentrationen im Bereich von 0,5 bis 15 % vorliegen.

## Revendications

1. Compositions pharmaceutiques topiques contenant comme principes actifs :
a) un agent chélatant
b) un tocophérol
c) un antibiotique de type aminoglycoside choisi parmi la gentamycine à une concentration comprise entre 0,1 et 1% p/p ou la nétilmycine ou l'amikacine en concentrations comprises entre 0,5 et 8%.

2. Compositions pharmaceutiques topiques selon la revendication 1, caractérisées en ce que l'agent chélatant est choisi parmi l'acide édétique ou ses sels, la déferoxamine et le dimercaprol (BAL).

3. Compositions pharmaceutiques topiques selon la revendication 1 ou 2, dans lesquelles l'agent chélatant est présent à des concentrations comprises dans l'intervalle allant de 0,05 à 1% p/p.

4. Compositions pharmaceutiques topiques selon l'une quelconque des revendications précédentes, caractérisées en ce que le tocophérol est choisi parmi la vitamine E (α-tocophérol) ou des tocophérols mixtes.

5. Compositions pharmaceutiques topiques selon la revendication 4, dans lesquelles les tocophérols sont présents en concentrations comprises dans l'intervalle allant de 0,5 à 15%.
